# EUROPÄISCHE PATENTANMELDUNG

(11) **EP 1 179 332 A2**
(43) Veröffentlichungstag der Anmeldung: **13.02.2002**
(21) Anmeldenummer: 01118999.0
(22) Anmeldetag: 06.08.2001
(51) Int. Cl.: A61H 35/00, A61H 35/02, A61F 9/00

(54) **Augenspülvorrichtung**

(30) Priorität: 11.08.2000 DE 10039448
(71) Anmelder: Benzinger, Kurt, Dr. med., 83623 Dietramszell (DE)
(72) Erfinder: Benzinger, Kurt, Dr. med., 83623 Dietramszell (DE)
(74) Vertreter: Störle, Christian, Dr.

(57) **Zusammenfassung**

Zum Spülen eines Auges ist eine Augenspülvorrichtung vorgesehen, die mit einem Spülflüssigkeit enthaltenden Behälter zu einem Augenspülgerät vereinigt werden kann. Die Augenspülvorrichtung hat ein Augenkörbchen, das einen zum Aufsetzen auf das zu spülende Auge ausgebildeten Ring aufweist, der von mehreren Armen gehalten ist, eine unterhalb der Oberkante des Ringes liegende Austrittsöffnung für die Spülflüssigkeit und ein die Austrittsöffnung speisendes Rohr, das starr ist und an seinem der Austrittsöffnung abgewandten Ende eine Spitze aufweist, mit der ein Verschlußstopfen des Behälters durchstoßen werden kann.

## Beschreibung

Die Erfindung betrifft eine Augenspülvorrichtung mit einem Augenkörbchen, das einen zum Aufsetzen auf das zu spülende Auge ausgebildeten Ring aufweist, der von mehreren Armen gehalten ist, einer unterhalb der Oberkante des Ringes liegenden Austrittsöffnung für Spülflüssigkeit und einem die Austrittsöffnung speisenden Rohr.

Bei Unfällen mit Schädigung der Augen durch Verätzung oder Einwirkung von Stäuben oder Aerosolen soll das betroffene Auge möglichst schnell gespült werden, um Schadstoffe rasch zu verdünnen und zu entfernen. Dazu werden neben frischem Leitungswasser auch isotone Salzlösungen (Natriumchlorid 0,9%, Ringerlösung, balanced salt solution), Pufferlösungen (Isogutt® ) (= 4,48%iger Natriumphosphatpuffer) oder chelatbildende Lösungen (Previn® ) empfohlen, wobei je nach Art des Unfalls und der das Auge verunreinigenden Substanz einer anderen Lösung der Vorzug zu geben ist. Zum Teil unterliegen diese Augenspüllösungen dem Arzneimittelrecht und sind nur über Apotheken zu beziehen. Darüber hinaus sollte die Spülflüssigkeit stets steril sein, so daß eine regelmäßige Überwachung der Spülflüssigkeit notwendig ist.

Für solche Spülungen sind ferner Augenduschen bekannt, die fest mit einem Leitungsnetz verbunden sind. Dies bietet den Vorteil, daß langanhaltend gespült werden kann. Damit verbunden ist allerdings der Nachteil, daß derartige Augenduschen aus Kostengründen meist nur zentral oder an wenigen Stellen verfügbar sind, so daß im Notfall erst weite Wege zurückzulegen sind und wertvolle Zeit verloren geht, zudem ist Leitungswasser nur bedingt geeignet.

In Labors, ärztlichen Praxen und in der Industrie sind Augenspülflaschen mit oder ohne Inhalt weit verbreitet. Ein Beispiel ist die von Tobin Scandinavia AB, Schweden, hergestellte und von der Firma wero-medical vertriebene Augenspülflasche. Eine ähnliche Augenspülflasche ist auch in der GB 561985 beschrieben. Diese Flaschen muß man entweder vor dem Spülen mit Leitungswasser füllen oder, falls sie bereits mit einer Spüllösung befüllt sind, überwachen und gegebenenfalls regelmäßig erneuern, um eine möglichst geringe Belastung mit Keimen zu gewährleisten. Darüber hinaus ist durch das manchmal nötige Nachfüllen ersterer eine Unterbrechung bei einer Spülung oft nicht zu vermeiden.

Aus der GB 2328876 A ist ein Medikamentensprühaufsatz zum Sprühen von Medikamenten auf die Hornhaut eines Auges bekannt, der auf einem Deckel sitzt, welcher auf ein Medizinfläschchen geschraubt werden kann.

Bei der Anwendung mancher Augenspülgeräte muß der Patient liegen, wodurch er unvermeidlich naß wird, da diese Geräte so ausgebildet sind, daß die Lösung nur per Schwerkraft ins Auge gelangen kann. Durch die dadurch bedingte, am Anfang sehr große psychische Hemmschwelle geht oft wertvolle Zeit verloren.

Möchte man unterschiedliche Spülflüssigkeiten zur Verfügung haben, so ist es nach dem Stand der Technik unumgänglich, entweder eine Augenspülflasche, die vor dem Spülen mit einer gewünschten Spülflüssigkeit befüllt werden muß, oder mehrere Augenspülflaschen mit den verschiedenen Spülflüssigkeiten vorzuhalten. Letzteres ist gerade vor dem Hintergrund der begrenzten Haltbarkeit und der erforderlichen Sterilität nachteilig.

Der Erfindung liegt deshalb die Aufgabe zugrunde, eine Augenspülvorrichtung zu schaffen, die größtmögliche Freiheit bei der Wahl der Spülflüssigkeit bietet, ohne daß mehrere Augenspülflaschen vorgehalten werden müssen oder ein Befüllvorgang erforderlich ist.

Diese Aufgabe wird bei einer Augenspülvorrichtung mit einem Augenkörbchen, das einen zum Aufsetzen auf das zu spülende Auge ausgebildeten Ring aufweist, der von mehreren Armen gehalten ist, einer unterhalb der Oberkante des Ringes liegenden Austrittsöffnung für Spülflüssigkeit und einem die Austrittsöffnung speisenden Rohr dadurch gelöst, daß das Rohr starr ist und an seinem der Austrittsöffnung abgewandten Ende eine Spitze aufweist.

Die Erfindung sieht also eine Augenspülvorrichtung vor, die auf einen Spülflüssigkeit enthaltenden Behälter aufgesetzt wird, so daß Augenspülvorrichtung und Behälter zusammen ein Augenspülgerät bilden. Durch die Gestaltung der Augenspülvorrichtung mit einem starren, die Austrittsöffnung speisenden Rohr, das am der Austrittsöffnung abgewandten Ende eine Spitze hat, kann die Augenspülvorrichtung besonders schnell und bequem auf die Spülflüssigkeit enthaltenden Behälter aufgesetzt werden, beispielsweise indem ein Verschlußpfropfen oder ein Septum, die den Behälter verschließen, mit der Spitze durchstochen wird.

So kann beispielsweise eine handelsübliche Plastik-Infusionsflasche zusammen mit der Augenspülvorrichtung zu einem vollwertigen Augenspülgerät werden. Dies bietet größtmögliche Freiheit bezüglich der Art der Spülflüssigkeit: Es ist eine nahezu beliebige Vielfalt an Augenspülflüssigkeiten möglich. Man muß lediglich mit geeigneten Flüssigkeiten gefüllte Behälter, beispielsweise Plastik-Infusionsflaschen, vorhalten. Als Spülflüssigkeiten können somit alle für die Augenspülung geeigneten Flüssigkeiten Einsatz finden. Besonders gute medizinische Erfolge erreicht man mit physiologisch unbedenklichen Infusionslösungen, insbesondere auch mit Pufferlösungen oder Kochsalz- bzw. Ringerlösungen.

Weiter ist ein schneller Behälterwechsel und damit eine nahezu ununterbrochene langanhaltende Spülung möglich.

Um ein betriebsfähiges Augenspülgerät zu erhalten, wird die erfindungsgemäße Augenspülvorrichtung auf einen geeigneten Behälter aufgesetzt, der Verschlußstopfen oder ein Verschlußseptum mit der scharfen Spitze des Augenspülgerätes durchstochen und das Rohr vollständig in den Behälter eingeführt. Die Länge des Rohres sollte natürlich an den Behälter geeignet angepaßt sein. Durch Erzeugen von Überdruck im Behälter, beispielsweise durch Kompression einer Plastikflasche, wird die im Behälter befindliche Spülflüssigkeit durch das Rohr zur Austrittsöffnung gefördert und sprüht von dort auf das zu spülende Auge, wenn das Augenkörbchen geeignet aufgesetzt ist.

Die Austrittsöffnung gibt die für die Spülung des Auges verwendete Spülflüssigkeit ab. Es ist deshalb besonders zweckmäßig, wenn die Austrittsöffnung, beispielsweise durch mehrere Einzellöcher so gestaltet ist, daß sie einen brauseartigen Strahl erzeugt.

Das Rohr dient dazu, ein Einführen in einen die Spülflüssigkeit enthaltenden Behälter zu ermöglichen. Soll dabei beispielsweise ein Verschlußstopfen durchstoßen werden, hat es sich als besonders zweckmäßig herausgestellt, wenn die Spitze eine schneidenden Anschliff aufweist. Wie die Spülflüssigkeit in das Rohr eintritt, ist für die Erfindung nicht wesentlich. Eine besonders vollständige Entleerung des Behälters erreicht man, wenn sich die Eintrittsöffnung in das Rohr unmittelbar in der Spitze befindet. Für Anwendungszwecke, bei denen beim Einführen des Rohres in den Behälter eine in der Spitze ausgebildete Eintrittsöffnung möglicherweise zugesetzt oder verstopft werden könnte, ist es zweckmäßig, die Eintrittsöffnung nicht in der Spitze sondern oberhalb davon auszubilden.

Durch die Ausgestaltung des Augenkörbchens zusammen mit dem starren Rohr ist ein verläßliches Fördern der Spülflüssigkeit zur Austrittsöffnung, ein zuverlässiges Spülen des Auges sowie ein sicherer Ablauf der benutzten Spülflüssigkeit durch das Körbchen nach unten erreicht, so daß die Spülung am sitzenden Patienten mit nach unten gewandtem Gesicht erfolgen kann. Dies hat nicht nur den Vorteil, daß der Patient beim Augenspülen nicht naß wird, sondern gleichzeitig wird durch die Haltung des nach unten gewandten Gesichtes ein schnelles Herausspülen von Verunreinigungen oder Fremdkörpern erreicht.

Das Augenkörbchen der Augenspülvorrichtung soll dafür sorgen, daß ein bestimmter Mindestabstand zwischen Austrittsöffnung der Spülflüssigkeit und zu spülendem Auge nicht unterschritten wird. Eine besonders gute Spülwirkung wird erreicht, wenn das Augenkörbchen so ausgebildet ist, daß es im aufgesetzten Zustand die Augenlider leicht spreizt, da dann der Spülstrahl den oberen und unteren Konjunktivalsack des Auges spülen kann.

Bei der Fortbildung des Augenkörbchens hat es sich als zweckmäßig erwiesen, dieses an anatomische und physiologische Gegebenheiten anzupassen. Mit einer längsovalen Form des Ringes sowie abgerundeten Profilen ohne Kanten oder Grate ist dies besonders gut erreicht.

Eine besonders kostengünstige Produktion läßt sich erreichen, wenn das Augenkörbchen aus weichem Kunststoff besteht, da dann beispielsweise Spritzgußverfahren für die Herstellung in Frage kommen. Eine besonders zweckmäßige Weiterbildung dieses Augenkörbchens erreicht man, wenn der aufzusetzende Ring von zwei Armen gehalten wird, die andererseits mit der Austrittsöffnung verbunden sind.

Das Material des Rohres ist prinzipiell frei wählbar, solange es starr ist. Dabei haben sich starre Kunststoffmaterialien als besonders günstige Materialien für das Rohr erwiesen. Als besonders robust hat sich auch ein Rohr aus einem gut fertigbaren, beispielsweise hartlötbaren Material herausgestellt, insbesondere ein Messingrohr.

Es hat sich allgemein gezeigt, daß ein kostengünstige Fertigung immer dann relativ einfach erreicht werden kann, wenn bereits vorhandene, massenproduzierte Standardteile eingesetzt werden. In einer besonders bevorzugten Weiterbildung der Augenspülvorrichtung wird deshalb ein bekanntes Infusionsüberleitungsgerät verwendet, wie es üblicherweise bei der Entnahme von Infusionslösung aus einer entsprechenden Infusionsplastikflasche Verwendung findet. Diese Infusionsüberleitungsgeräte weisen üblicherweise eine Sichtkammer auf, an deren Unterteil eine sogenannte Mandrinspitze zum Anschluß an die Plastikflasche gebildet ist und an dessen Oberseite ein Schlauch angeschlossen ist, über den die Infusionslösung entnommen wird. Ein solches Infusionsüberleitungsgerät kann nun zur Herstellung einer erfindungsgemäßen Augenspülvorrichtung verwendet werden, wenn am Oberteil der Sichtkammer die Austrittsöffnung der Augenspülvorrichtung angebracht wird, und am Unterteil das starre Rohr. Zusätzlich kann eine die Sichtkammer überbrückende Verbindung eingesetzt werden, die das Rohr mit der Austrittsöffnung verbindet, da ansonsten erst die gesamte Sichtkammer mit Spülflüssigkeit gefüllt werden müßte, bis Spülflüssigkeit aus der Austrittsöffnung austritt.

Die Erfindung wird nachfolgend unter Bezugnahme auf die Zeichnung in Ausführungsbeispielen näher erläutert. In der Zeichnung zeigt:
- Fig. 1: eine erste Ausführungsform einer Augenspülvorrichtung;
- Fig. 2: eine zweite Ausführungsform einer Augenspülvorrichtung mit Bestandteilen eines Infustionsüberleitungsgerätes;
- Fig. 3: eine Draufsicht auf eine mögliche Ausführungsform eines Augenkörbchens;
- Fig. 4: eine dritte Ausführungsform einer Augenspülvorrichtung;
- Fig. 5: eine vierte Ausführungsform einer Augenspülvorrichtung mit starrem Rohr und Kunststoffaugenkörbchen; und
- Fig. 6: eine Schnittdarstellung durch ein Rohr einer Augenspülvorrichtung.

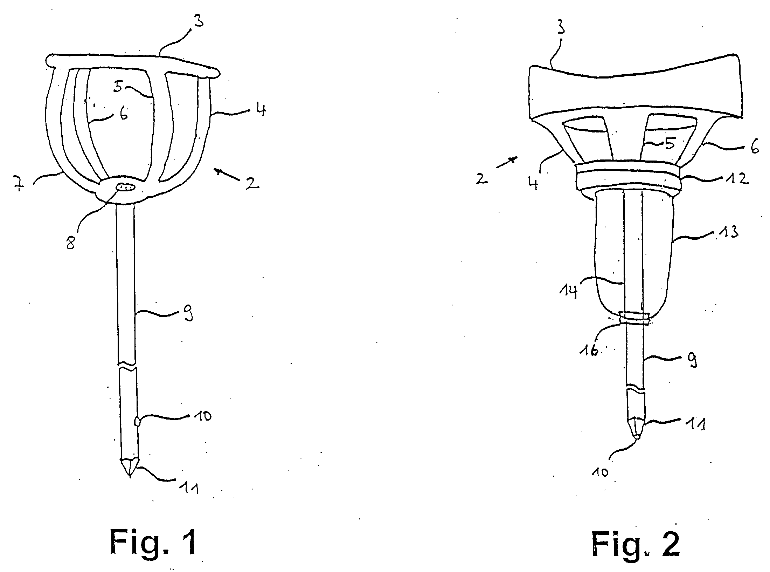

In Fig. 1 ist ein Augenspülvorrichtung gezeigt, die zum Einstecken in eine Plastikflasche, die die Spüllösung enthält, ausgebildet ist. Diese Augenspülvorrichtung weist ein Augenkörbchen 2 auf, an dessen Unterseite ein starres Rohr 9 befestigt ist, das in einer Spitze 11 mit Anschliff endet. Nahe der Spitze befindet sich eine Eintrittsöffnung 10, in der Spülflüssigkeit in das Rohr 9 eintreten kann. Das Rohr 9 endet in einer Austrittsöffnung 8 am Boden des Augenkörbchens. Die Austrittsöffnung 8 hat mehrere Löscher, so daß dem Rohr an der Eintrittsöffnung 10 zugeführte Spülflüssigkeit an der Austrittsöffnung 8 mit einem brauseartigen Strahl austritt.

Das Augenkörbchen 2 hat einen Ring 3 mit längsovaler Form. Setzt man den Ring 3 auf das zu spülende Auge auf, sorgt der Ring 3 dafür, daß die Augenlider gespreizt und vom Augenkörbchen 2 in dieser Stellung festgehalten werden. Der Ring 3 ist mit der am Boden des Augenkörbchens 2 befindlichen Austrittsöffnung 8 über vier Arme 4, 5, 6 und 7 verbunden.

Das Rohr 9 ist starr und besteht aus einem Kunststoffmaterial, ebenso der Ring 3 sowie die Arme 4, 5, 6, 7, wobei aber der Ring 3 sowie die Arme 4, 5, 6, 7 aus einem im Vergleich zum Rohr 9 weicheren, flexibleren und somit für den Patienten auf der Haut angenehmeren Kunststoff besteht. Durch die Öffnungen zwischen den Armen 4, 5, 6, 7 ist der Ablauf benutzter Spülflüssigkeit möglich, wenn die Spülung am sitzenden Patienten mit nach unten gewandten Gesicht erfolgt. Die Spitze 11 mit schneidendem Anschliff ist dafür geeignet, den Verschlußstopfen einer Plastikflasche zu durchstoßen.

Durch Kompression der Flasche wird dann Spüllösung der Eintrittsöffnung 10 unter Druck zugeführt, steigt im Rohr 9 an und tritt als brauseartiger Strahl an der Austrittsöffnung 8 aus. Die Luftzufuhr zur Flasche, d. h. der Ausgleich für die entnommene Spülflüssigkeit, erfolgt durch Regurgitation über das Rohr 9. Dabei strömt Luft durch das Rohr 9 von der Austrittsöffnung 8 zur Eintrittsöffnung 10.

Eine weitere Ausführungsform der Augenspülvorrichtung ist in Fig. 2 dargestellt. Hier sitzt das Augenkörbchen 2 auf dem Unterteil eines Infusionsüberleitungsgerätes, das starr ist. Das in diesem Fall aus einem weichen Kunststoff bestehende Augenkörbchen 2 weist ebenfalls einen Ring 3 auf, der von vier Armen 4, 5, 6 (der vierte Arm ist in der perspektivischen Darstellung der Fig. 2 nicht zu sehen) gehalten und mit der (in der perspektivischen Darstellung ebenfalls nicht zu sehen) Austrittsöffnung verbunden ist. Die Austrittsöffnung befindet sich in einer Flanschverbindung 12, mit der das Augenkörbchen 3 mit dem Unterteil eines Infusionsüberleitungsgerätes verbunden ist. Das Augenkörbchen 3 besteht aus flexibel-zähem Kunststoffmaterial. Der Ring 3 hat ebenfalls längsovale Form mit abgerundeten Profilen ohne Kanten oder Grate.

Das Unterteil des Infusionsüberleitungsgerätes besteht aus dem Unterteil der Sichtkammer 13 (die aber, falls es erwünscht ist, auch weggelassen werden kann) sowie dem als Rohr 9 dienenden Mandrinstab, in dessen Spitze 11 sich die Eintrittsöffnung 10 befindet. Die Länge des Rohres 9 ist bei der Ausführungsform der Fig. 2 ebenso wie bei der beschriebenen Ausführungform der Fig. 1 an die Tiefe der Plastikflasche, auf der die Augenspülvorrichtung aufgesetzt werden soll, angepaßt.

Am Verbindungsstück 16, an dem das Rohr 9 mit dem Unterteil der Sichtkammer 13 verbunden ist, weisen viele Infusionsüberleitungsgeräte eine Belüftungsöffnung auf. Da diese für die Augenspülvorrichtung keine Funktion hat, wird man sie zweckmäßigerweise blind verschließen oder sogar ganz weglassen. In die Darstellung der Fig. 2 ist sie der Übersichtlichkeit halber nicht eingezeichnet.

Hat man die Augenspülvorrichtung nun auf eine Plastikflasche gesetzt und somit ein funktionsfertiges Augenspülgerät erhalten, füllt sich bei Druck auf die Plastikflasche zuerst die Sichtkammer 13. Erst bei weiterem Druck tritt Flüssigkeit aus der Austrittsöffnung 8 aus. Möchte man dies vermeiden, ist es zweckmäßig, die Sichtkammer 13 mittels eines Förderrohrs 14 zu überbrücken, das einerseits am Verbindungsstück 16 angeschlossen ist, an dem das Rohr 9 in das Unterteil der Sichtkammer 13 mündet, und andererseits mit der Austrittsöffnung 8 verbunden ist. Diese Variante ist in Fig. 2 dargestellt.

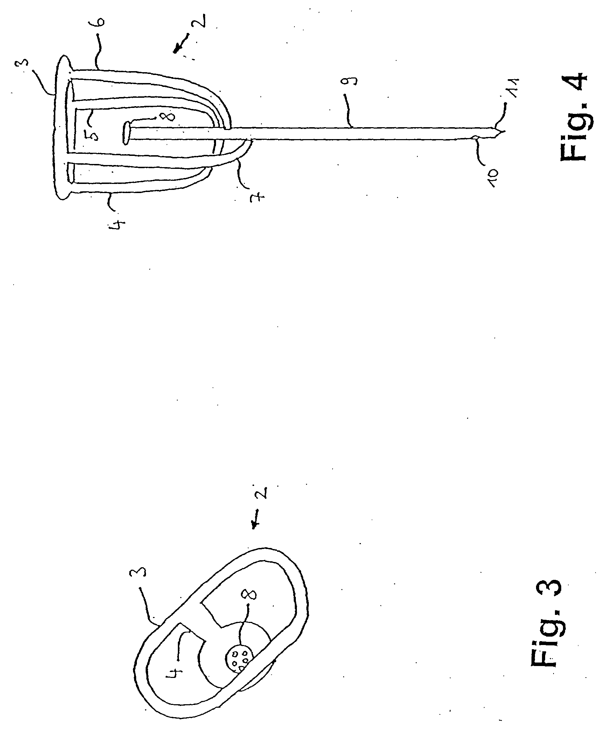

Das Augenkörbchen 2 ist in den Ausführungsformen der Fig. 1 und Fig. 2 mit einem Ring 3 versehen, der von vier Armen 4, 5, 6, 7 getragen wird. Es ist jedoch auch möglich, diese Zahl der Arme geringer zu wählen, wobei man einen etwaigen Stabilitätsverlust nötigenfalls durch breitere Arme ausgleichen kann. Ein solches Augenkörbchen 2 ist in perspektivischer Darstellung in Fig. 3 gezeigt. Es weist ebenfalls einen Ring 3 auf, der allerdings nur über zwei Arme 4, 5 (in Fig. 3 ist aufgrund der perspektivischen Darstellung nur ein Arm zu sehen) mit der Austrittsöffnung 8 verbunden ist.

In Fig. 4 ist eine weitere Augenspülvorrichtung gezeigt, bei der der Abstand der Austrittsöffnung 8 zur Oberkante des Ringes 3 geringer ist als bei den bisherigen Ausführungsformen. Darüber hinaus ist bei dieser Ausführungsform eine höhere Stabilität dadurch erreicht, daß die Arme 4, 5, 6, 7, die den Ring 3 am Rohr 9 halten, in unterschiedlichen Höhen am Rohr 9 angebracht sind. Dadurch ergibt sich zum einen ein Produktionsvorteil, da beispielsweise bei einem hartlötbaren Material, z. B. Messing, Lötverbindungen leichter ausgeführt werden können, zum anderen wird höhere Stabilität erreicht, da ein eventuelles Biegemoment nicht nur in einem schmalen axialen Bereich des Rohres 9 wirkt.

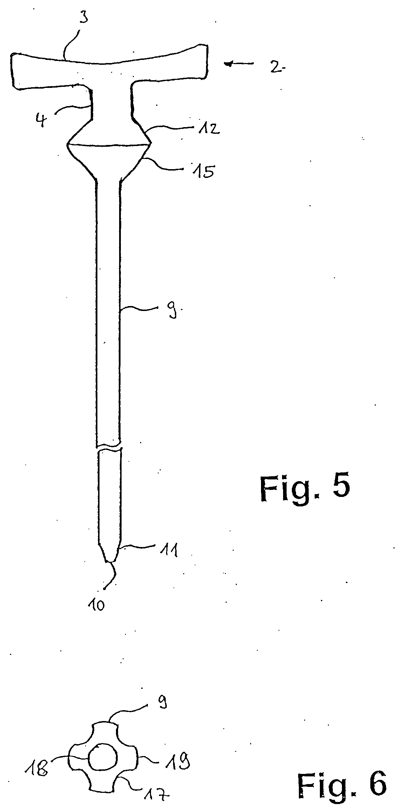

In Fig. 5 ist eine weitere Ausführungsform gezeigt, bei der ein Rohr 9 aus Messing mit einem Augenkörbchen 2 aus weichem Kunststoff kombiniert ist. Das Rohr 9, das wiederum eine Spitze 11 sowie eine Eintrittsöffnung 10 aufweist, hat an seinem oberen Ende eine Aufweitung 15, mit der die Flanschverbindung 12 an der Unterseite des Augenkörbchens 2 verbunden ist, beispielsweise durch eine Klebung. Diese Variante vereinigt die Vorzüge eines sehr starren Rohres 9 mit den physiologischen Vorteilen eines flexiblen, elastischen Augenkörbchens 2. In Fig. 5 ist dargestellt, daß der Ring 3 wiederum von zwei Armen 4 getragen wird, jedoch ist dies nur beispielhaft.

Das starre Rohr 9 ist besonders stabil, wenn es mit dem in Fig. 6 dargestellten Querschnitt versehen ist. Der Förderkanal 18 hat in dieser Ausführungsform einen Durchmesser zwischen 2 mm und 3 mm, der Außendurchmesser des mit Längsnuten 17 versehen Rohrs 9 liegt zwischen 8 mm und 10 mm. Durch die Längsnuten 17 ergeben sich stabilisierende Vorsprünge 19, die sich axial entlang des Rohres 9 erstrecken. Durch diese Gestaltung kann auch ein entsprechendes Kunststoffmaterial zur Ausbildung des starren Rohres 9 verwendet bzw. der Durchmesser des Rohres 9 entsprechend abgesenkt und dennoch die nötige Stabilität erreichen werden.

## Patentansprüche

1. Augenspülvorrichtung mit
- einem Augenkörbchen (2), das einen zum Aufsetzen auf das zu spülende Auge ausgebildeten Ring (3) aufweist, der von mehreren Armen (4, 5, 6, 7) gehalten ist,
- einer unterhalb der Oberkante des Ringes (3) liegenden Austrittsöffnung (8) für Spülflüssigkeit und
- einem die Austrittsöffnung (8) speisenden Rohr (9),
**dadurch gekennzeichnet, daß**
- das Rohr (9) starr ist und an seinem der Austrittsöffnung (8) abgewandten Ende eine Spitze (11) aufweist.

2. Vorrichtung nach Anspruch 1, **dadurch gekennzeichnet, daß** das Rohr (9) am der Austrittsöffnung (8) abgewandten Ende eine Eintrittsöffnung (10) aufweist, die oberhalb der Spitze (11) liegt.

3. Vorrichtung nach Anspruch 1 oder 2, **dadurch gekennzeichnet, daß** das Augenkörbchen (2) aus elastischem Kunststoff besteht und daß zwei Arme (4, 5) vorgesehen sind, die an ihrem dem Ring (3) abgewandten Ende mit der Austrittsöffnung (8) verbunden sind.

4. Vorrichtung nach einem der Ansprüche 1 bis 3, **dadurch gekennzeichnet, daß** das Rohr (9) aus einem Metall besteht.

5. Vorrichtung nach einem der Ansprüche 1 bis 4, **dadurch gekennzeichnet, daß** die Austrittsöffnung (8) so gestaltet ist, daß sie einen brauseartigen Strahl erzeugt.

6. Vorrichtung nach einem der Ansprüche 1 bis 5, **dadurch gekennzeichnet, daß** die Spitze (11) einen schneidenden Anschliff aufweist.

7. Vorrichtung nach einem der Ansprüche 1 bis 6, **dadurch gekennzeichnet, daß** die Arme (4, 5, 6, 7) fest mit der Austrittsöffnung (8) und mit einer Sichtkammer (13) eines Infusionsüberleitungsgerätes verbunden sind, wobei in die Sichtkammer (13) ein Förderrohr (14) eingesetzt ist, das das am Unterteil der Sichtkammer (13) befestigte Rohr (9) mit der Austrittsöffnung (8) verbindet.
